# FASCICULE DE BREVET EUROPEEN

(11) **EP 1 373 164 B1**
(45) Date de publication et mention de la délivrance du brevet: **20.05.2009**
(21) Numéro de dépôt: 02732792.3
(22) Date de dépôt: 04.04.2002
(51) Int. Cl.: C04B 38/04, B22F 3/11, G01B 5/06, G01N 19/00, C04B 35/447, A61F 2/28

(54) **PROCEDE DE CONTROLE DE LA COALESCENCE DES PARTICULES FORMANT UN EDIFICE POREUX PAR EXEMPLE UN IMPLANT OSSEUX**
VERFAHREN ZUR STEUERUNG DER KOALESZENZ VON TEILCHEN, DIE EINE PORÖSE STRUKTUR BILDEN, BEISPIELSWEISE EIN KNOCHENIMPLANTAT
METHOD FOR CONTROLLING COALESCENCE OF PARTICLES FORMING A POROUS STRUCTURE FOR EXAMPLE A BONE IMPLANT

(30) Priorité: 05.04.2001 FR 0104658
(43) Date de publication de la demande: 02.01.2004
(73) Titulaire: Biocetis, 62600 Berck (FR)
(72) Inventeur: DESCAMPS, Michel, F-59135 Wallers (FR); HARDOUIN, Pierre, F-62520 Le Touquet (FR); LU, Jianxi, F-13000 Marseille (FR); MONCHAU, Francine, F-59264 Onnaing (FR)
(74) Mandataire: Duthoit, Michel Georges André
(86) Numéro de dépôt international: PCT/FR2002/001183
(87) Numéro de publication internationale: WO 2002/081408

(56) Documents cités:
- EP-A- 0 263 489
- WO-A-92/06653
- WO-A-95/21053
- WO-A-98/34654
- DD-A- 301 493
- US-A- 3 941 529
- US-A- 4 371 484
- US-A- 4 628 042
- US-A- 4 717 115
- US-A- 5 458 837
- US-A- 5 549 123

## Description

L'invention concerne un procédé de contrôle de la coalescence de particules, un procédé et un dispositif de fabrication d'un édifice de particules pontées entre elles mettant en oeuvre un tel procédé de contrôle ainsi qu'un procédé d'obtention d'un produit à pores interconnectés, notamment destinés à la substitution osseuse, utilisant l'édifice obtenu selon le procédé de fabrication précédent.

Bien que plus particulièrement destiné à la fabrication d'un implant osseux, ledit procédé d'obtention pourra toutefois être utilisé pour la fabrication de tous types de produits poreux.

Actuellement, dans le domaine de la substitution osseuse, il est connu d'utiliser des implants à pores interconnectés.

Leur fabrication s'effectue de la façon suivantes :
- réalisation d'un édifice de particules pontées entre elles grâce à un traitement de coalescence pour constituer avec les particules dudit édifice une charpente occupant l'emplacement des pores de l'implant que l'on souhaite obtenir,
- remplissage des interstices présents entre les particules de l'édifice avec la matière de l'implant,
- élimination de la charpente.

Toutefois, la porosité des implants obtenus n'est pas toujours satisfaisante. En effet, celle-ci est uniquement déterminée par le temps de traitement employé lors de la coalescence des particules de la charpente et les autres opérations de vérification, quand elles existent, sont effectuées, après fabrication, sur échantillons.

Il est notamment connu du document US 3,880,971 un procédé de contrôle de retrait d'un produit en céramique à base d'aluminium pendant le fritage ou densification.

Il est par ailleurs connu du document US 5,174,933 un procédé de fabrication d'un moule fermé constitué des matrices, comprenant des senseurs afin de mesurer la variation de pression et la variation de déplacement relatif des matrices.

Le but de l'invention est de proposer un procédé de contrôle de la coalescence de particules qui pallie les inconvénients précités et offre une meilleure fiabilité.

Un autre but de l'invention est de proposer un procédé de contrôle de la coalescence de particules qui soit non destructif.

Un autre but de l'invention est de proposer un procédé de contrôle de la coalescence de particules qui soit effectué en ligne.

D'autres buts et avantages de l'invention apparaîtront au cours de la description qui va suivre qui n'est donnée qu'à titre indicatif et qui n'a pas pour but de la limiter.

L'invention concerne tout d'abord un procédé de contrôle de la coalescence des particules formant un édifice soumis à un traitement destiné à entraîner le pontage desdites particules entre elles, procédé dans lequel on relève au moins une grandeur physique, dont l'évolution est mesurable en continu et à laquelle le pontage des particules est corrélé, et on provoque l'arrêt du traitement lorsque ladite grandeur physique a atteint une valeur donnée correspondant à la coalescence souhaitée, ladite grandeur physique étant le retrait dudit édifice.

Selon l'invention, ledit procédé est caractérisé par le fait que l'on obtient la coalescence des particules par dilution chimique, grâce à un solvant dont on imprègne ledit édifice puisque l'on élimine partiellement de façon à laisser un film mince de solvant sur lesdites particules.

L'invention concerne également un procédé de fabrication d'un édifice de particules pontées entre elles dans lequel on empile lesdites particules selon la forme désirée pour ledit édifice, on fait subir auxdites particules un traitement permettant leur coalescence et, lors dudit traitement, on contrôle la coalescence des particules selon le procédé précédent.

L'invention concerne enfin un procédé d'obtention d'un produit à pores interconnectés, notamment destiné à la substitution osseuse, dans lequel :
- on élabore un édifice de particules pontées entre elles, selon le procédé de fabrication défini plus haut pour constituer avec les particules dudit édifice une charpente occupant l'emplacement des pores du produit que l'on souhaite obtenir, lesdites particules présentant entre elles des interstices,
- on comble lesdits interstices avec une matière destinée à constituer ledit produit,
- on fait disparaître ladite charpente.

L'invention sera mieux comprise à la lecture de la description suivante qui en illustre certains modes de réalisation.

L'invention concerne tout d'abord un procédé de contrôle de la coalescence des particules formant un édifice soumis à un traitement destiné à entraîner le pontage desdites particules entre elles.

Il pourra s'agir de particules de formes quelconques ou prédéterminées, telles que sphériques ou tubulaires. La dimension de ces particules sera ajustée en fonction de l'application désirée. Dans le cas de la fabrication d'édifices destinés à l'obtention d'implants osseux, leurs tailles pourront varier de 50 micromètres à 5 millimètres, voire plus. Dans un même édifice, lesdites particules pourront être de mêmes dimensions ou non.

Pour contrôler leur coalescence, on relève au moins une grandeur physique, dont l'évolution est mesurable en continu et à laquelle le pontage des particules est corrélé, et on provoque l'arrêt du traitement lorsque ladite grandeur physique a atteint une valeur donnée correspondant à la coalescence souhaitée.

Selon l'invention, on mesure à titre dedite grandeur physique le retrait dudit édifice. On a en effet constater que ce retrait est directement corrélé au pontage entre les particules. Plus précisément, il est proportionnel à celui-ci et ne dépend pas des conditions extérieures, ce qui facilite l'établissement des relations de corrélation et l'exploitation des mesures.

On peut ainsi mieux contrôler le phénomène de pontage et obtenir des édifices dont les interconnexions entre particules pourront être supérieures à la moitié des dimensions de celles-ci.

Ladite mesure du retrait pourra être effectuée, par exemple, par mesure de l'évolution de l'épaisseur de l'édifice selon une direction donnée.

L'invention concerne également un procédé de fabrication d'un édifice de particules pontées entre elles dans lequel on empile lesdites particules selon la forme désirée pour ledit édifice et on fait subir auxdites particules un traitement permettant leur coalescence et, lors dudit traitement, on contrôle la coalescence des particules selon le procédé précédent, ceci afin de mieux maîtriser les phénomènes de pontage grâce à la grandeur physique relevée, à savoir le retrait de l'édifice.

Selon un mode de réalisation particulier, la coalescence des particules sera obtenue par dilution chimique. Plus précisément, on pourra réaliser une imprégnation dudit édifice dans un solvant, puis éliminer partiellement ledit solvant après un certain temps en laissant subsister un film mince de solvant sur lesdites particules afin que celui-ci se localise au point de contact entre les particules et permette le pontage. Il pourra s'agir d'un solvant, notamment cétonique, permettant une dissolution lente et progressive desdites particules.

On peut noter qu'avec ce mode de traitement, l'établissement des interconnexions entre particules sera influencé, notamment, par l'agressivité chimique du solvant par rapport aux particules, par le temps de trempage de l'édifice dans le solvant, par la quantité de solvants utilisée ou encore la température ambiante. Ainsi, par exemple, pour des particules de même granulométrie, plus la température ambiante sera faible, plus le temps nécessaire pour obtenir un niveau donné d'interconnexion sera long.

Par contre, comme déjà dit, pour des particules de même granulométrie, un retrait donné correspond à un niveau d'interconnexion donné, quelles que soient les conditions extérieures. Il n'est donc pas nécessaire de reproduire, lors du pontage d'un édifice donné les conditions dans lesquelles la relation de corrélation entre le retrait de l'édifice et la coalescence des particules pour des édifices de ce type aura été établie.

Dans le cas d'un traitement par dilution chimique, les particules employées pourront être, par exemple, les produits de type polystyrène, polyéthylène, polyamide, polyméthylmétacrylate ou autres.

Toujours selon ce mode de traitement, l'arrêt du phénomène de pontage sera provoqué par élimination du solvant résiduel, par exemple par séchage de l'édifice.

L'invention concerne encore un dispositif de fabrication d'un édifice de particules pontées entre elles, notamment pour la mise en oeuvre du procédé précédent.

Ledit dispositif comprend un moule, apte à autoriser un empilement desdites particules et à subir un traitement de coalescence de celles-ci.

Dans le cas d'un traitement par dilution chimique, le moule sera constitué, par exemple, d'une matière inerte chimiquement par rapport aux solvants utilisés.

Selon l'invention, ledit moule est muni de moyens de mesure du retrait de l'empilement de particules. Il permet ainsi un contrôle de leur coalescence lors de leur traitement.

Lesdits moyens de mesure sont constitués, par exemple :
- d'un piston destiné à coulisser dans le moule selon une direction donnée, au contact desdites particules,
- d'un capteur des déplacements dudit piston.

On mesure ainsi le retrait de l'édifice par mesure de la variation de son épaisseur selon ladite direction. Le capteur employé pourra être du type mécanique, électrique, optoélectronique ou autres.

Plus précisément, ledit moule pourra comprendre un couvercle fermant une cavité de moulage apte à accueillir lesdites particules, ledit couvercle constituant le piston desdits moyens de mesure. Le capteur de ces derniers sera alors constitué, notamment, d'une tige assujettie audit couvercle et de moyens de détection des mouvements de ladite tige.

L'invention concerne encore un procédé d'obtention d'un produit à pores interconnectés, dans lequel :
- on élabore un édifice de particules pontées entre elles selon le procédé de fabrication défini plus haut pour constituer avec les particules dudit édifice une charpente occupant l'emplacement des pores du produit que l'on souhaite obtenir, lesdites particules présentant entre elles des interstices,
- on comble lesdits interstices avec une matière destinée à constituer ledit produit,
- on fait disparaître ladite charpente.

On peut ainsi, grâce au contrôle de la coalescence des particules de l'édifice, contrôler la porosité de l'implant, ceci de manière plus fiable qu'auparavant, de façon non destructive et sans opération de reprise. Il est de la sorte possible d'obtenir des produits présentant une architecture dans laquelle les pores soient contrôlés tant en uniformité, qu'en dimensions ou en morphologie.

Dans le cas de la fabrication d'implants osseux, on pourra combler les interstices présents entre les particules formant l'édifice avec une suspension constituée de poudre minérale ou métallique finement divisée dans un solvant aqueux ou organique incluant divers composés tels que, dispersants, liants, agents anti-mousse, agents mouillants et/ou autres.

Les poudres minérales ou métalliques seront choisies, notamment, parmi les composés bio-compatibles tels que bioverres, composés à base de phosphates de calcium purs ou dopés (hydroxyapatite, phosphate tricalcique), les carbonates de calcium purs ou dopés, les oxydes d'aluminium ou de zirconium purs ou dopés, te titane, les aciers inoxydables purs ou dopés et/ou autres.

On laisse alors sécher ladite suspension avant l'élimination de la charpente qui pourra être réalisée par traitement thermique.

Après élimination de la charpente, on pourra également envisager des opérations de densification ou de frittage de la matière restante dont les températures seront adaptées aux matériaux employés. Ces opérations pourront être réalisées sous air ou sous atmosphères contrôlées.

Naturellement, d'autres modes de mise en oeuvre, à la portée de l'homme de l'art, auraient pu être envisagés sans pour autant sortir du cadre de l'invention.

## Revendications

1. Procédé de contrôle de la coalescence des particules formant un édifice soumis à un traitement destiné à entraîner le pontage desdites particules entre elles, procédé dans lequel on relève au moins une grandeur physique, dont l'évolution est mesurable en continu et à laquelle le pontage des particules est corrélé, et on provoque l'arrêt du traitement lorsque ladite grandeur physique a atteint une valeur donnée correspondant à la coalescence souhaitée, ladite grandeur physique étant le retrait dudit édifice, ledit procédé étant **caractérisé par le fait que** l'on obtient la coalescence des particules par dilution chimique, grâce à un solvant dont on imprègne ledit édifice puis que l'on élimine partiellement de façon à laisser un film mince de solvant sur lesdites particules et dans lequel la mesure du retrait s'effectue par mesure de l'évolution de l'épaisseur de l'édifice selon une direction donnée.

2. Procédé de fabrication d'un édifice de particules, pontées entre elles, dans lequel on empile lesdites particules selon la forme désirée pour ledit édifice, on fait subir auxdites particules ledit traitement permettant leur coalescence et, lors dudit traitement, on contrôle la coalescence des particules selon le procédé de la revendication 1.

3. Procédé de fabrication d'un édifice de particules selon la revendication 2, dans lequel on provoque l'arrêt du traitement par élimination du solvant résiduel par séchage.

4. Procédé d'obtention d'un produit à pores interconnectés, notamment destiné à la substitution osseuse, dans lequel :
- on élabore un édifice de particules pontées entre elles selon le procédé de fabrication défini à l'une quelconque des revendications 2 à 3 pour constituer avec les particules dudit édifice une charpente occupant l'emplacement des pores du produit que l'on souhaite obtenir, lesdites particules présentant entre elles des interstices,
- on comble lesdits interstices avec une matière destinée à constituer ledit produit,
- on fait disparaître ladite charpente.

## Claims

1. A method for controlling the coalescence of the particles forming an edifice subjected to a treatment intended for bridging said particles relative to one another, a method wherein at least one physical quantity is observed, whereof the evolution is measurable continuous and to which the bridging of the particles is correlated, and the treatment is stopped when said physical quantity has reached a given value corresponding to the coalescence requested, said physical quantity being the retraction from said edifice, said method being **characterised in that** the coalescence of the particles is obtained by chemical dilution, thanks to a solvent with which said edifice is impregnated since it is eliminated partially so as to leave a thin film of solvent on said particles and wherein the retraction is assessed by measuring the thickness of the edifice along a given direction.

2. A method of manufacture of an edifice of particles, bridged to one another, wherein said particles are piled up according to the shape desired for said edifice, said particles are subjected to said treatment enabling their coalescence and, during said treatment, the coalescence of the particles is controlled according to the method of claim 1.

3. A method of manufacture of an edifice of particles according to claim 2, wherein the treatment is stopped by eliminating the residual solvent by drying.

4. A method for obtaining a product with interconnected pores, in particular intended for bone substitution, wherein:
- an edifice of particles bridged relative to one another is prepared according to the method of manufacture defined in any of the claims 2 to 3 to form with the particles of said edifice a structure occupying the location of the pores of the intended product, said particles exhibiting interstices therebetween,
- said interstices are filled in with a material intended to form said product,
- said structure is removed.

## Patentansprüche

1. Verfahren zur Kontrolle der Koaleszenz der Partikeln, die eine Struktur bilden, die einer Behandlung unterzogen wird, vorgesehen, um die Vernetzung der besagten Partikel untereinander zu bewirken, seiend dies ein Verfahren, bei welchem zumindest eine physikalische Größe, deren Verlauf kontinuierlich messbar ist und mit welcher die Vernetzung der Partikel korreliert ist, aufgezeichnet wird und die Unterbrechung der Behandlung ausgelöst wird, wenn die besagte physikalische Größe einen bestimmten Wert erreicht hat, der der gewünschten Koaleszenz entspricht, wobei die besagte physikalische Größe die Schrumpfung der besagten Struktur sei, wobei das besagte Verfahren **dadurch gekennzeichnet** sei, dass die Koaleszenz der Partikel durch chemische Verdünnung dank einem Lösungsmittel erhalten wird, mit welchem die besagte Struktur imprägniert wird, welches anschließend zum Teil beseitigt wird, derart, um auf den besagten Partikeln einen Lösungsmitteldünnschichtfilm fortbestehen zu lassen, und bei welchem die Messung der Schrumpfung durch die Messung des Verlaufs der Dicke der Struktur nach einer bestimmten Richtung erfolgt.

2. Verfahren zur Herstellung einer Struktur von Partikeln, die untereinander vernetzt sind, bei welchem die besagten Partikeln nach der für die besagte Struktur gewünschten Form aufgeschichtet werden, wobei die besagten Partikeln der besagten Behandlung unterzogen werden, die ihre Koaleszenz erlaubt, und während der besagten Behandlung die Koaleszenz der Partikeln nach dem Verfahren von Anspruch 1 kontrolliert wird.

3. Verfahren zur Herstellung einer Struktur von Partikeln nach Anspruch 2, bei welchem die Unterbrechung der Behandlung durch Beseitigung des restlichen Lösungsmittels durch Trocknung ausgelöst wird.

4. Verfahren zur Herstellung eines Produkts mit untereinander verbundenen Poren, insbesondere vorgesehen für die Substitution von Knochen, bei welchem:
- eine Struktur aus untereinander vernetzten Partikeln nach dem Herstellungsverfahren nach irgendeinem der Ansprüche 2 bis 3 hergestellt wird, um mit den Partikeln der besagten Struktur ein Gerüst zu bilden, das den Platz der Poren des zu erhaltenden Produkts besetzt, wobei die besagten Partikeln untereinander Zwischenräume aufweisen,
- die besagten Zwischenräume mit einem Material gefüllt werden, das vorgesehen ist, um das besagte Produkt zu bilden,
- das besagte Gerüst entfernt wird.
